# EUROPÄISCHE PATENTSCHRIFT

(11) **EP 1 259 481 B1**
(45) Veröffentlichungstag und Bekanntmachung des Hinweises auf die Patenterteilung: **26.10.2011**
(21) Anmeldenummer: 01900139.5
(22) Anmeldetag: 08.01.2001
(51) Int. Cl.: C07C 279/22, C07D 233/64, C07D 249/02, A61K 31/41, A61P 9/10

(54) **SUBSTITUIERTE BENZOYLGUANIDINE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**
SUBSTITUTED BENZOYLGUANIDINES, METHOD FOR THEIR PRODUCTION, THEIR USE AS A MEDICAMENT OR DIAGNOSTIC AGENT AND A MEDICAMENT CONTAINING THE SAME
BENZOYLGUANIDINES SUBSTITUEES, LEUR PROCEDE DE PRODUCTION, LEUR UTILISATION EN TANT QUE MEDICAMENT OU AGENT DIAGNOSTIQUE ET MEDICAMENT LES CONTENANT

(30) Priorität: 19.01.2000 DE 10001879
(43) Veröffentlichungstag der Anmeldung: 27.11.2002
(73) Patentinhaber: Sanofi-Aventis Deutschland GmbH, 65929 Frankfurt am Main (DE)
(72) Erfinder: WEICHERT Dr., Andreas G., 22391 Hamburg (DE); ALBUS, Udo, 61197 Florstadt (DE); JANSEN, Hans-Willi, 65527 Niedernhausen (DE); KLEEMANN, Heinz-Werner, 65474 Bischofsheim (DE); LANG, Hans-Jochen, 65719 Hofheim (DE)
(74) Vertreter: Michalski Hüttermann & Partner Patentanwälte
(86) Internationale Anmeldenummer: PCT/EP2001/000137
(87) Internationale Veröffentlichungsnummer: WO 2001/053256

(56) Entgegenhaltungen:
- EP-A- 0 556 673
- EP-A- 0 640 588
- EP-A- 0 640 593
- EP-A- 0 699 663
- EP-A- 0 699 666
- EP-A- 0 758 644
- EP-A- 0 794 172
- EP-A- 0 810 207
- EP-A- 0 814 077

## Beschreibung

Die Erfindung betrifft Benzoylguanidine der Formel I 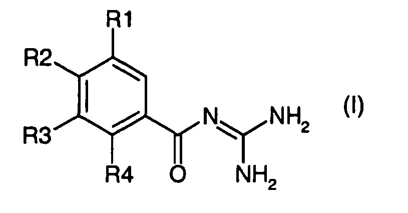 worin bedeuten:
- R1: CF₃;
- R2: -Y-p-(C₆H₄)- R11, -Y-m-(C₆H₄)- R11 oder -Y-o-(C₆H₄)- R11;
- R11: Imidazolyl, oder Triazolyl, das jeweils unsubstituiert oder substituiert ist mit 1 bis 2 Substituenten ausgewählt aus der Gruppe bestehend aus F, Cl, CF₃, CH₃, Methoxy, Dimethylamino und Benzyl;
- Y: Sauerstoff;
- R3: Wasserstoff;
- R4: Methyl,
sowie deren pharmazeutisch verträgliche Salze.

Die Erfindung betrifft weiterhin ein Verfahren zur Herstellung der Verbindung I, **dadurch gekennzeichnet, dass** man eine Verbindung der Formel II 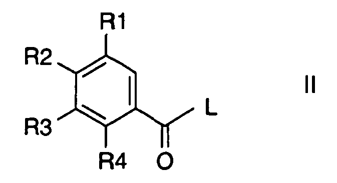 worin R1 bis R4 die angegebene Bedeutung besitzen und L für eine leicht nucleophil substituierbare Fluchtgruppe steht,
mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie die Methylester der Formel II mit L = OCH₃ durch Behandeln mit gasförmigem HCl. in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluorbor at ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 2, 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zinkverbindungen.

Benzoylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Den Verbindungen I ähnliche Verbindungen sind bekannt aus der Europäischen Offenlegungsschrift 640 593 (HOE 93/F 220 ). Diese enthalten jedoch in der Position von R4 (ortho- Stellung) stets andere Substituenten; die erfindungsgemäßen sind dort weder erwähnt noch nahegelegt.

Gegenüber den bekannten Verbindungen zeichnen sich die erfindungsgemäßen Verbindungen durch eine außerordentlich hohe Wirksamkeit in der Inhibition des Na⁺/H⁺-Austauschs aus, sowie durch eine verbesserte Wasserlöslichkeit aus

Sie haben ebenso wie die bekannten Verbindungen keine unerwünschten und nachteiligen salidiuretischen, jedoch sehr gute antiarrhythmische Eigenschaften, wie sie beispielsweise zur Behandlung von Krankheiten wichtig sind, die bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielsweise des allergischen, cardiogenen, hypovolämischen und des bakteriellen Schocks. Darüber hinaus zeichnen sich die erfindungsgemäßen Verbindungen der Formel I durch starke inhibierende Wirkung auf die Proliferationen von Zellen, beispielsweise der Fibroblasten- Zellprolifera:ion und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielsweise der essentiellen Hypertonie, geeignet.

Es wurde außerdem gefunden, daß Verbindungen der Formel I eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken.. Es wurde nun gefunden, daß Verbindungen der Formel I bezüglich der Beeinflussung der Serumlipidspiegel wertvolle therapeutisch verwertbare Eigenschaften zeigen. So erniedrigen sie signifikant die erhöhten Serum Konzentration von LDL und VLDL, wie sie beispielsweise durch erhöhte dietätische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die Verbindungen der Formel I zu einer deutlichen Reduktion der durch Stoffwechselanomalien induzierten Infarkte und insbesondere zu einer signifikanten Verminderung der induzierten Infarktgröße und dessen Schweregrades. Weiterhin führen Verbindungen der Formel I zu einem wirksamen Schutz gegen durch Stoffwechselanomalien induzierte Endothelschädigungen. Mit diesem Schutz der Gefäße gegen das Syndrom der endothelialen Dysfunktion sind Verbindungen der Formel I wertvolle Arzneimittel zur Prävention und zur Behandlung koronarer Gefäßsspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter cardialer Hypertrophien und Cardiomyopathien, zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)- Hemmern und Angiotensin- Rezeptorantagonisten, eine Kombination eines NHE- Inhibitors der Formel I mit einem blutfettspiegelsenkenden Wirkstoff, bevorzugt mit einem HMG-CoA-Reduktaseinhibitor (z. B. Lovastatin oder Pravastatin), wobei letzterer eine hypolipidämische Wirkung herbeiführt und dadurch die hypolipidämischen Eigenschaften des NHE- Inhibitors der Formel steigern, erweist sich als günstige Kombination mit verstärkter Wirkung und vermindertem Wirkstoffeinsatz.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositorien- Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine. Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den. Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise mindestens 0,01 mg/kg, insbesondere mindestens 0,1 mg/kg bis höchstens 10 mg/kg, vorzugsweise höchstens 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i. v. Anwendung, etwa bei einem Infarktpatienten auf der Intensivstation können bis zu 200 mg pro Tag notwendig werden.

### Liste der Abkürzungen:

- CDI: Carbonyldiimidazol
- MeOH: Methanol
- DMF: N,N-Dimethylformamid
- RT: Raumtemperatur
- EE: Ethylacetat (EtOAc)
- eq: Äquivalent
- ES: Elektrospray-lonisation

### Experimenteller Teil

### Beispiel 1

### 4-[(lmidazol-1-yl)-phenoxy]-2-methyl-5-trifluormethyl-benzoylguanidin-dihydrochlorid, farbloser Feststoff, M⁺+H (ES) = 404.

Syntheseweg:
a) 4-[(Imidazol-1-yl)-phenoxy]-2-methyl-5-trifluormethyl-benzoesäuremethylester durch Reaktion von 4-Fluor-2-methyl-3-trifluormethyl-benzoesäuremethyl-ester mit 1 eq 4-(Imidazol-1-yl)-phenol in Gegenwart von 4 eq Kaliumkarbonat in DMF bei 120°C innerhalb von 16 h. Nach Evaporation des Lösungsmittels wird wässerig aufgearbeitet und mit EE ausgeschüttelt. Nach Trocknung des LM wird evaporiert, farbloses Öl, M⁺ (ES) = 376.
b) 4-[(lmidazol-1-yl)-phenoxy]-2-methyl-5-trifluormethyl-benzoesäure durch basische Hydrolyse mit einem Überschuß an 2N NaOHaq in MeOH bei RT innerhalb 2 h. Nach Azidifizieren mit 2N HCl folgt Extraktion mit EE, nach Trocknen des LM und Evaporation fällt ein farbloses Öl an, M⁺ (ES) = 362.
   4-[(Imidazol-1-yl)-phenoxy]-2-methyl-5-trifluormethyl-benzoylguanidin-dihydrochlorid durch Aktivierung mit 2 eq CDI in DMF und anschließender Reaktion mit 6 eq Guanidiniumhydrochlorid in Gegenwart von 7 eq Diisopropyl-ethylamin bei RT innerhalb von 3 h. Nach Entfernen des LM erfolgt eine präparative HPLC (CH₃CN/H₂O), anschließende Salzbildung mit etherischer Salzsäure.

### Beispiel 2: 4-(Triazol-1-yl)-phenoxy-2-methyl-3-trifluormethyl-benzoylguanidin-bis trifluoracetat, farbloser Feststoff, M⁺+H (ES) = 405.

Syntheseweg:
a) 4-[(Triazol-1-yl)-phenoxy]-2-methyl-5-trifluormethyl-benzoesäuremethylester analog 1 a) durch Reaktion mit 1 eq 4-(Triazol-1-yl)-phenol,
   farbloses Öl, M⁺ (ES) = 377.
b) 4-[(Triazol-1-yl)-phenoxy]-2-methyl-5-trifluormethyl-benzoesäure analog 1 b),
   farbloses Öl, M⁺ (ES) = 363.
c) 4-[(Triazol-1-yl)-phenoxy]-2-methyl-5-trifluormethyl-benzoylguanidin-bis trifluoracetat analog 1 c), jedoch Salzbildung mittels Trifluoressigsäure.

## Patentansprüche

1. Benzoylguanidine der Formel I 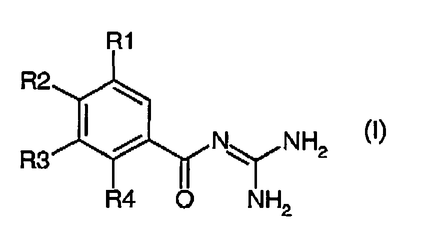
R1 CF₃;
R2 -Y-p-(C₆H₄)- R11, -Y-m-(G₆H₄)- R11 oder -Y-o-(C₆H₄)- R11;
R11 Imidazolyl oder Thiazolyl, das jeweils unsubstituiert oder substituiert ist mit 1 bis 2 Substituenten ausgewählt aus der Gruppe bestehend aus F, Cl, CF₃, CH₃, Methoxy, Dimethylamino und Benzyl;
Y Sauerstoff;
R3 Wasserstoff;
R4 Methyl,
sowie deren pharmazeutisch verträgliche Salze.

2. Verfahren zur Herstellung einer Verbindung der Formel 1, **dadurch gekennzeichnet, dass** man eine Verbindung der Formel II 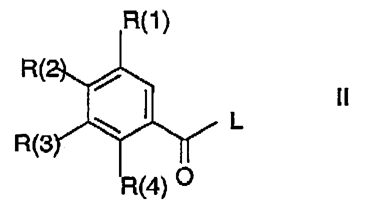 worin R1 bis R4 die angegebene Bedeutung besitzen und L für eine leicht nucleophil substituierbare Fluchtgruppe steht,
mit Guanidin umsetzt.

3. Verbindungen der Formel I nach Anspruch 1 zur Behandlung oder zur Prophylaxe von durch ischämische Zustände verursachten Krankheiten, zur Behandlung von Schockzuständen, zum Einsatz bei chirurgischen Operationen und Organtransplantationen und zur Konservierung und Lagerung von Transplantaten für chirurgische Maßnahmen.

4. Verwendung einer Verbindung I nach Anspruch 1 zur Herstellung eines Medikaments zu Behandlung oder Prophylaxe von durch ischämische Zustände verursachten Krankheiten.

5. Verwendung einer Verbindung I nach Anspruch 1 zur Herstellung eines Medikaments zur Behandlung oder Prophylaxe des Herzinfarkts und von Arrhythmien.

6. Verwendung einer Verbindung I nach Anspruch 1 zur Herstellung eines Medikaments zur Behandlung oder Prophylaxe der Angina Pectoris.

7. Verwendung einer Verbindung I nach Anspruch 1 zur Herstellung eines Medikaments zur Behandlung oder Prophylaxe von ischämischen Zuständen des Herzens.

8. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

9. Verwendung einer Verbindung I nach Anspruch 1 zur Herstellung eines Medikaments zur Behandlung oder Prophylaxe von ischämischen Zuständen peripherer Organe und Gliedmaßen.

10. Verwendung einer Verbindung I nach Anspruch 1 zur Herstellung eines Medikaments zur Behandlung von Schockzuständen.

11. Verwendung einer Verbindung I nach Anspruch 1 zur Herstellung einer. Medikaments zum Einsatz bei chirurgischen Operationen und Organtransplantationen.

12. Verwendung einer Verbindung I nach Anspruch 1 zur Herstellung eines Medikaments zur Konservierung und Lagerung von Transplantaten für chirurgische Maßnahmen.

13. Arzneimittel, enthaltend eine wirksame Menge einer Verbindung I nach Anspruch 1.

## Claims

1. A benzoylguanidine of the formula I 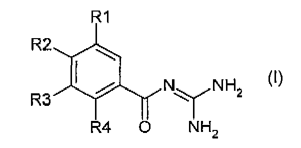
R1 is CF₃;
R2 is -Y-p-(C₆H₄)-R11, -Y-m-(C₆Hg)-R11 or -X-o-(C₆Hg)-R11;
R11 is imidazolyl or triazolyl which is in each case unsubstituted or substituted by 1 to 2 substituents selected from the group consiting of F, Cl, CF₃, CH₃, methoxy, dimethylamino and benzyl;
Y is oxygen;
R3 is hydrogen;
R4 is methyl;
as well as pharmaceutically acceptable salts thereof.

2. A process for the preparation of a compound of the formula I, which comprises reacting a compound of the formula II 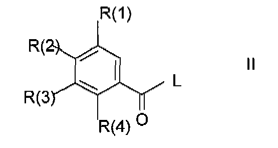 in which R1 to R4 have the meaning given and L is a leaving group which can readily be substituted nucleophilically,
with guanidine.

3. A compound of the formula I as claimed in claim 1 for the treatment or for the prophylaxis of diseases caused by ischemic conditions, for the treatment of conditions of shock, for employment in surgical operations and organ transplantations and for the presentation and storage of transplants for surgical measures.

4. The use of a compound I as claimed in claim 1 for the production of a medicament for the treatment or prophylaxis of diseases caused by ischemic conditions.

5. The use of a compound I as claimed in claim 1 for the production of a medicament for the treatment or prophylaxis of cardiac infarction and of arrhythmias.

6. The use of a compound I as claimed in claim 1 for the production of a medicament for the treatment or prophylaxis of angina pectoris.

7. The use of a compound I as claimed in claim 1 for the production of a medicament for the treatment or prophylaxis of ischemic conditions of the heart.

8. The use of a compound I as claimed in claim 1 for the production of a medicament for the treatment or prophylaxis of ischemic conditions of the peripheral and central nervous system and of stroke.

9. The use of a compound I as claimed in claim 1 for the production of a medicament for the treatment or prophylaxis of ischemic conditions of peripheral organs and limbs.

10. The use of a compound I as claimed in claim 1 for the production of a medicament for the treatment of conditions of shock.

11. The use of a compound I as claimed in claim 1 for the production of a medicament for employment in surgical operations and organ transplantations.

12. The use of a compound I as claimed in claim 1 for the production of a medicament for the preservation and storage of transplants for surgical measures.

13. A medicament, comprising an effective amount of a compound I as claimed in claim 1.

## Revendications

1. Benzoylguanidines de la formule I 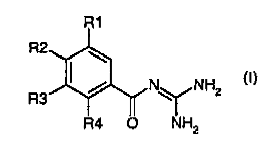 dans laquelle
R1 CF₃ ;
R2 -Y-p-(C₆H₄)-R11, -Y-m-(C₆H₄)-R11 ou -Y-o-(C₆H₄)-R11;
R11 imidazolyle ou triazolyle, qui est chaque non substitué ou substitué avec 1 à 2 substituants sélectionnés dans le groupe composé de F, Cl, CF₃, CH₃, méthoxy, diméthylamino et benzyle;
Y oxygène
R3 hydrogène
R4 méthyle
ainsi que leurs sels compatibles au niveau pharmaceutique.

2. Procédé de fabrication d'un composé de la formule I, **caractérisé en ce que** l'on convertit un composé de la formule II 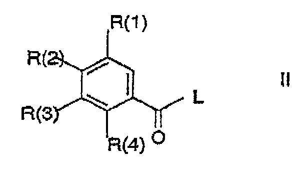 dans laquelle R1 à R4 ont la signification indiquée et L est un groupe partant à substitution nucléophile légère,
avec la guanidine.

3. Composés de la formule I selon la revendication 1 pour le traitement ou pour la prophylaxie de maladies provoquées par des états ischémiques, pour le traitement d'états de choc, pour utilisation dans des opérations chirurgicales et des transplantations d'organes et pour la conservation et le stockage d'organes à transplanter en vue de mesures chirurgicales.

4. Utilisation d'un composé I selon la revendication 1 pour la fabrication d'un médicament pour le traitement ou la prophylaxie de maladies provoquées par des états ischémiques.

5. Utilisation d'un composé I selon la revendication 1 pour la fabrication d'un médicament pour le traitement ou la prophylaxie de l'infarctus du myocarde et de l'arythmie.

6. Utilisation d'un composé I selon la revendication 1 pour la fabrication d'un médicament pour le traitement ou la prophylaxie de l'angine de poitrine.

7. Utilisation d'un composé I selon la revendication 1 pour la fabrication d'un médicament pour le traitement ou la prophylaxie d'états ischémiques du coeur.

8. Utilisation d'un composé I selon la revendication 1 pour la fabrication d'un médicament pour le traitement ou la prophylaxie d'états ischémiques du système nerveux périphérique et central et de l'apoplexie.

9. Utilisation d'un composé I selon la revendication 1 pour la fabrication d'un médicament pour le traitement ou la prophylaxie d'états ischémiques d'organes périphériques et de membres.

10. Utilisation d'un composé I selon la revendication 1 pour la fabrication d'un médicament pour le traitement d'états de choc.

11. Utilisation d'un composé I selon la revendication 1 pour la fabrication d'un médicament à utiliser lors d'opérations chirurgicales et de transplantations d'organes.

12. Utilisation d'un composé 1 selon la revendication 1 pour la fabrication d'un médicament pour la conservation et le stockage d'organes à transplanter en vue de mesures chirurgicales.

13. Médicament, contenant une quantité active d'un composé 1 selon la revendication 1.
